(19) Europäisches Patentamt

European Patent Office

Office européen des brevets

(11) **EP 1 721 597 A1**

(12) **EUROPÄISCHE PATENTANMELDUNG**

(43) Veröffentlichungstag:
**15.11.2006 Patentblatt 2006/46**

(51) Int Cl.:
*A61K 8/02* (2006.01)          *A61F 13/15* (2006.01)
*D04H 1/42* (2006.01)

(21) Anmeldenummer: **06004886.5**

(22) Anmeldetag: **10.03.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **09.05.2005   DE 102005022577**

(71) Anmelder: **Paul Hartmann AG**
**89522 Heidenheim (DE)**

(72) Erfinder:
• **Römpp, Angela**
**73119 Zell u.A. (DE)**
• **Mangold, Rainer, Dr.**
**89542 Herbrechtlingen (DE)**

(74) Vertreter: **Friz, Oliver**
**Patentanwälte,**
**Dreiss, Fuhlendorf, Steimle & Becker,**
**Postfach 10 37 62**
**70032 Stuttgart (DE)**

(54) **Mehrfach auf sich selbst gefaltetes Watteband**

(57)     Die Erfindung betrifft ein mehrfach auf sich selbst gefaltetes Watteband aus Baumwollfasern und/ oder Viskosefasern oder anderen zellulosischen Fasern mit einem Anteil von 3 - 80 Gew.-%, insbesondere von 3 - 50 Gew.-% Mikrostapelfasern, wobei das Watteband eine vertikale Steighöhe für eine wässrige Flüssigkeit von höchstens 40 mm aufweist.

Fig. 1b

EP 1 721 597 A1

**Beschreibung**

Die Erfindung betrifft ein mehrfach auf sich selbst gefaltetes Watteband.

Bekannte Z-förmig oder leporelloförmig auf sich selbst gefaltete Wattebänder sind aus 100% Baumwollfasern gebildet. Bekannte Wattebänder sind im Hinblick auf die Optimierung der Parameter der Weichheit und der Reinigungswirkung, die einander gegensinnig beeinflussen, verbesserungswürdig. Werden derartige Wattebänder bei verschiedensten Anwendungen zusammen mit einem flüssigen Reinigungsprodukt verwendet, so besteht bei derartigen dicken Produkten oftmals das Problem, dass das flüssige Reinigungsprodukt in das Innere des Wattebands gesaugt wird und daher mengenmäßig nicht optimal für die Reinigung zur Verfügung steht.

Der Erfindung liegt die Aufgabe zugrunde, ein Watteband hinsichtlich der vorgenannten Aspekte zu optimieren.

Diese Aufgabe wird erfindungsgemäß durch ein Watteband mit den Merkmalen des Anspruchs 1 gelöst.

Der Zusatz von Mikrostapelfasern, vorzugsweise einer Länge von wenigstens 5, insbesondere von wenigstens 7 mm innerhalb des beanspruchten mengenmäßigen Bereichs führt zu einer Verbesserung der Reinigungswirkung, ohne die Weichheit des Produkts spürbar herabzusetzen. Außerdem ist mit dem Zusatz von Mikrostapelfasern eine Verbesserung der Bauschelastizität und damit des Rückstellvermögens des Watteprodukts verbunden, was sich für die Griffigkeit und das Abreinigungsvermögen nach Druckbelastung sehr positiv auswirkt, indem nach einem Wischvorgang abgereinigte Bestandteile durch das verbesserte Rückstellvermögen besser im Inneren des Watteprodukts aufgenommen werden können. Als ganz besonders vorteilhaft erweist es sich insbesondere bei hohen Flächengewichten des Wattebands, dass die vertikale Flüssigkeitsaufnahme, für die als Maß die vertikale Steighöhe verwendet werden kann, höchstens 40 mm beträgt, da dies eine nicht übermäßige Flüssigkeitsabsorption in das Innere des Watteprodukts bedeutet, was als vorteilhaft angesehen werden kann, da flüssige Reinigungsprodukte länger an der Oberfläche des Watteprodukts zur Verfügung stehen. Die vertikale Steighöhe beträgt vorzugsweise 10 - 40 mm, insbesondere 15 - 38 mm.

Vorzugsweise werden in dem erfindungsgemäßen Watteband Mikrostapelfasern in einem Gewichtsanteil von 8 - 30% bezogen auf das Gesamtgewicht des Wattebandes eingesetzt.

Wenn vorstehend von Mikrostapelfasern die Rede ist, so werden hierunter synthetische Fasern einer Faserstärke von $\leq$ 1 dtex verstanden. Der Begriff der Mikrostapelfasern bringt dabei zum Ausdruck, dass für die Herstellung der Faservlieslage des Wattepads zuvor in einem separaten Herstellungsverfahren gebildete Mikrofasern einer bestimmten Länge oder eines bestimmten Längenbereichs verwendet werden.

Die Faserlänge der verwendbaren Mikrostapelfasern beträgt vorzugsweise 10 - 38 mm, insbesondere 15 - 32 mm.

Bei den Mikrostapelfasern kann es sich in weiterer Ausbildung der Erfindung um Polyester- (PES) oder um Viskosefasern handeln. Vorzugsweise werden Polyesterfasern, insbesondere von 0,9 dtex mit einer Faserlänge von ca. 18 mm eingesetzt.

Bei den eingesetzten Baumwollfasern handelt es sich vorteilhafterweise um Baumwollkämmlinge. Das Flächengewicht des Wattebands im flachgelegten Zustand beträgt vorzugsweise wenigstens 400 g/m$^2$, es beträgt vorteilhafterweise 400 g/m$^2$ - 800 g/m$^2$, insbesondere 450 g/m$^2$ - 750 g/m$^2$, und weiter insbesondere 500 g/m$^2$ - 650 g/m$^2$.

Seine Dichte beträgt vorteilhafterweise 0,01 - 0,1 g/m$^3$, insbesondere 0,02 - 0,07 g/m$^3$ und weiter insbesondere 0,03 - 0,06 g/m$^3$.

Wie bereits erwähnt, erweist es sich als vorteilhaft, wenn die vertikale Steighöhe 10 - 40 mm, insbesondere 15 - 38 mm beträgt.

Des Weiteren erweist es sich als besonders vorteilhaft, dass die Absinkdauer (Drahtkörbchenmethode) in wässriger Flüssigkeit wenigstens 3 s, insbesondere wenigstens 3,5 s, insbesondere wenigstens 4 s, und weiter insbesondere wenigstens 4,5 s beträgt.

Das Wasserhaltevermögen (Drahtkörbchenmethode) beträgt wenigstens 21 g/g, insbesondere wenigstens 24 g/g.

Es erweist sich als besonders vorteilhaft, dass das Rückstellvermögen des Wattebands, welches nach der nachfolgenden Testmethode bestimmt wird, größer als 50 % ist, insbesondere vorzugsweise wenigstens 52 % beträgt.

Zusätzlich können wärmeschmelzbare Bindefasern enthalten sein, vorzugsweise zu 5 - 20 Gew.-%, weiter bevorzugt 5 - 15 Gew.-% bezogen auf das Gesamtgewicht des Wattebandes.

In weiterer Ausbildung der Erfindung kann es sich bei den Bindefasern um Mehrkomponentenfasern, insbesondere Bikomponenten-Fasern handeln mit einer höher schmelzenden Trägerkomponente und einer niedriger schmelzenden Komponente.

Die Mehrkomponentenfasern, insbesondere Bikomponentenfasern, haben in vorteilhafter Weise eine Faserstärke von 1,3 - 10 dtex, insbesondere von 1,3 - 3 dtex und eine Faserlänge von 3 bis 60 mm. Vorteilhafterweise kommen Kern/Mantel-Fasern oder Seite-an-Seite-Fasern zum Einsatz.

Es hat sich als vorteilhaft erwiesen, wenn Bikomponentenfasern mit einem Copolyester (CO-PES) als niedrig schmelzender Komponente und Polyester (PES) als höher schmelzender Komponente verwendet werden.

In weiterer Ausbildung ist der Schmelzpunkt der wärmeschmelzbaren Bindefasern oder der niedrig schmelzenden Komponente (z.B. CO-PES)der Mehrkomponentenfasern geringer als der Schmelzpunkt der Mikrostapelfasern. Bspw. könnten Mikrostapelfasern aus einem Polyestermaterial verwendet werden mit einem Schmelzpunkt von etwa 256°C und CO-PES/PES-Bikomponentenfasern mit einem Schmelzpunkt der niedrig schmelzenden Komponente CO-PES von 110°C und der höher schmelzenden Komponente PES von 255°C. Solchenfalls könnte eine thermische Verfestigung des Faservlieses durchgeführt werden, ohne die höher schmelzende Komponente der Bikomponentenfasern und die Mikrostapelfasern thermisch zu verändern.

Bestimmung der vertikalen Steighöhe und Flüssigkeitsaufnahme:

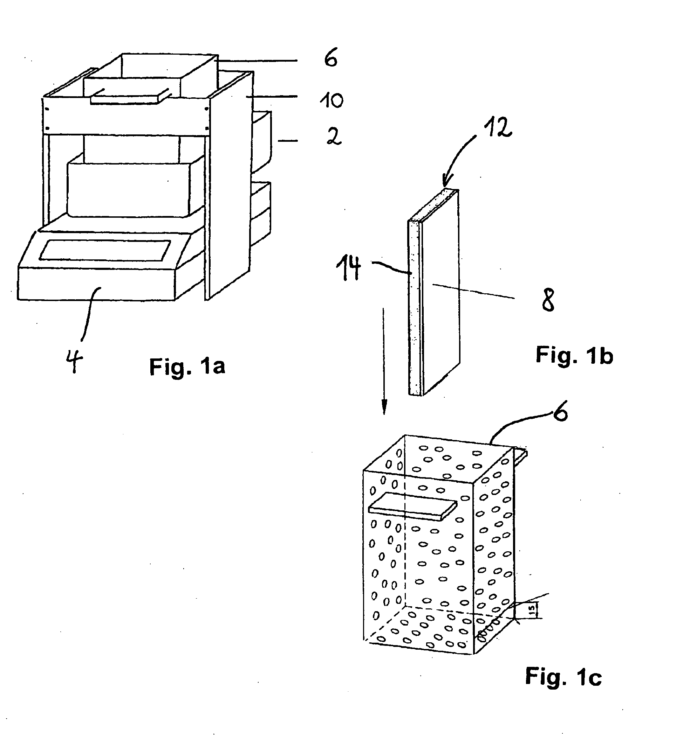

Zur Ermittlung der Flüssigkeitsaufnahmefähigkeit entgegen der Schwerkraft (vertikale Flüssigkeitsaufnahme), die im Ergebnis in $g_{Flüssigkeit}/g_{Watte}$ angegeben wird, wird ein Probenstreifen eines zuvor entfalteten Wattebands einer Größe von 40 x 100 mm mittig aus dem Watteband mit der längeren Abmessung in Längsrichtung des Wattepads präpariert. Dieser Probenstreifen wird durch eine Fixierplatte gehalten und gestützt und in ein Flüssigkeitsbad (0,9% NaCl-Lösung in demineralisiertem Wasser) etwa 15 mm tief vertikal eingetaucht, und es wird dann die vertikal, also entgegen der Schwerkraft, infolge der "Dochtwirkung" des Wattebands aus der Flüssigkeit nach oben gesogene Flüssigkeit gemessen. Dieser Test ist angelehnt an Edana 10.3-99.
Es wird hierfür ein ausreichend großer Wasserbehälter 2 auf einer Waage 4 positioniert (s. Figuren 1 a,b,c). Ein Probenhalter 6 wird so bezüglich Wasserbehälter 2 und Waage 4 positioniert, dass die Fixierplatte 8 (noch ohne Watteband) 15 mm in die Prüflösung eintaucht. In diesem Zustand wird die Waage 4 tariert. Es wird dann der Probenhalter 6, der im dargestellten Fall der Figur 1 a auf einem Ständergestell 10 ruht, abgenommen, damit der Prüfkörper 12 aus Probenstreifen 14 und Fixierplatte 8 an dem Probenhalter 6 angeordnet werden kann. Der Probenstreifen 14 (nur Watte ohne Fixierplatte) wurde zuvor auf 0,1 g genau gewogen. Der Probenhalter 6 wird dann mit dem Prüfkörper 12 wieder in genau dieselbe Position gebracht. Die Prüflösung kann jetzt in den Probenstreifen 14 des Prüfkörpers 12 eindringen. Nach zehn Minuten wird die vertikal aufgesogene Flüssigkeitsmenge in Form einer Gewichts- bzw. Massenreduzierung an der Waage in Gramm abgelesen (vertikale Flüssigkeitsaufnahme). Die spezifische vertikale Flüssigkeitsaufnahme wird dann in g/g angegeben.
Nach dem Ablesen der aufgenommenen Flüssigkeitsmenge wird der Prüfkörper 12 aus der Flüssigkeit entnommen, und es wird die Steighöhe, bis zu der die Flüssigkeit aufgestiegen ist, abzüglich der 15 mm Eintauchtiefe visuell bestimmt und mit einem Lineal auf einen mm genau gemessen.

Bestimmung des Wasserhaltevermögens und Absinkdauer :

Der folgende Test ist beschrieben in PH.EUR.1997, Monografie Verbandwatte aus Baumwolle; es handelt sich um einen Test der Saugfähigkeit unter Bestimmung der Absinkdauer eines mit Fasermaterial befüllten Drahtkörbchens in einer Flüssigkeit und des Wasserhaltevermögens. Das hierzu verwendbare Drahtkörbchen ist ein zylindrischer Korb aus Kupferdraht mit einem Drahtdurchmesser von 0,4 mm. Die Höhe beträgt 80 mm, der Durchmesser 50 mm, die Maschenweite 15 - 20 mm und die Masse 2,7 +/- 0,3 g. Ferner findet ein Becherglas mit Durchmesser 11 - 12 cm Verwendung.
Zu testendes Fasermaterial in abgelegter Vliesform wird in einer Prüfmenge von 5 g in das Drahtkörbchen eingelegt. Zuvor wurde der Korb auf 0,01 g genau gewogen (M1). Die 5 g Probenmaterial bilden die Masse M2. Das Becherglas wird mit demineralisiertem Wasser bis zu einer Höhe von etwa 100 mm gefüllt und der gefüllte Korb wird aus einer Höhe von 10 mm auf das Wasser fallen gelassen. Mit einer Stopuhr wird die Zeit bis zum Absinken unter die Wasseroberfläche bestimmt, also die Absinkdauer. Unmittelbar nach der Bestimmung der Absinkdauer wird der Korb aus dem Wasser gehoben und zum Abtropfen 30 s lang in seiner Längsachse horizontal gehalten. Nach Ablauf der Abtropfzeit wird der Korb in ein tariertes Becherglas (M3) gegeben und auf 0,01 g genau gewogen (M4).
Das Wasserhaltevermögen ergibt sich in g/g zu

$$\frac{M4 - (M2 + M3)}{M2 - M1}$$

Die Absinkdauer und das Wasserhaltevermögen werden als Mittelwert aus zumindest drei Bestimmungen angegeben. Sie lassen sich durch Veränderung des Anteils absorbierender Fasern (Baumwoll- oder andere cellulosische Fasern) und/oder durch Zusatz von Hydrophilierungsmittel einstellen.

Bestimmung der Dichte des Wattebands:

Zur Bestimmung der Dichte des Wattebands wird entsprechend DIN 53885 vorgegangen. Es wird ein Prüfling einer vorbekannten Fläche, etwa ca. 20 cm² gefertigt und dessen Masse auf 0,1 mg genau bestimmt. Es wird dann unter Vorgabe einer Belastung von 2 g/cm² die Dicke unter Verwendung eines Dickenmessgeräts mit einer gegenüber der Probengröße größeren Messfläche auf 0,01 mm genau gemessen. Die Dichte ergibt sich dann aus Masse des Prüflings dividiert durch Fläche dividiert durch Dicke. Sie wird auf zwei Dezimalen gerundet in g/cm³ angegeben.

Bestimmung des Rückstellvermögens:

Die nachfolgende Messmethode entspricht bzw. ist angelehnt an DIN 54305 vom Februar 1976. Die Methode dient der Bestimmung des druckelastischen Verhaltens von Faservliesstoffen, indem deren Zusammendrückbarkeit und die darauffolgende Rückstellung ermittelt werden.

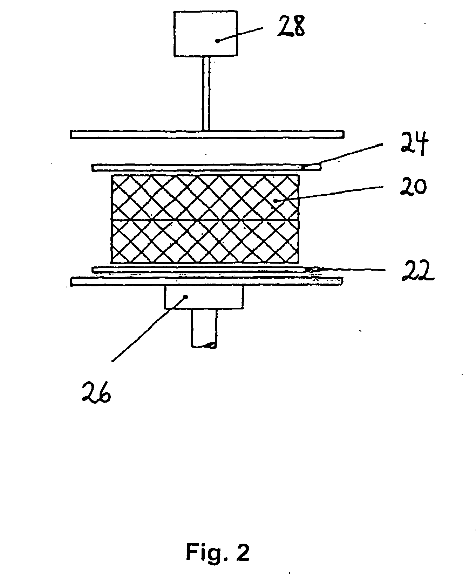

Hierfür werden vorliegend aus dem entfalteten und flachgelegten Watteband Einzelproben von 100 mm x 100 mm Abmessung ausgestanzt. Durch einfaches Übereinanderschichten der Einzelproben wird ein Stapel von mindestens 50 mm Höhe gebildet. Beim Übereinanderschichten wird jede Einzelprobe gegenüber der vorherigen um 90° verdreht angeordnet. Der so gebildete Stapel wird nachfolgend als "Messprobe" 20 bezeichnet (Figur 2). Es wird dann die Masse M der Messprobe 20 durch Wiegen bestimmt. Aus dieser Masse der 100 x 100 mm großen übereinandergestapelten Prüflinge (Messprobe) kann durch Multiplikation mit 100 und Division durch die Anzahl n der Prüflinge das Flächengewicht des hierfür verwandten Wattebands in g/m² errechnet werden, also

$$\frac{M \cdot 100}{n} \quad \text{in } g/m^2$$

Als Prüfeinrichtung wird beispielsweise eine Zwick Universalprüfmaschine Typ 1425 verwendet. Das Prinzip für die Messung des Rückstellvermögens ist anhand der Fig. 2 schematisch dargestellt. Die Messprobe 20 wird auf eine Unterlageplatte 22 gelegt, und auf die Messprobe, also auf den Stapel, wird eine Vorlastplatte 24 einer Abmessung von ca. 110 mm x 110 mm und eines Gewichts von ca. 30 g $\pm$ 2 g aufgelegt (s. Figur 2). In diesem Zustand, also unter Aufliegen der Vorlastplatte 24 einer Masse von ca. 30 g, wird die Dicke der Messprobe 20 bestimmt, indem an allen vier Ecken der Vorlastplatte 24 gemessen wird. Aus den vier Messungen wird der Mittelwert gebildet, er wird als $a_{30}$-Dicke bezeichnet.

Die mittig auf die Prüfeinrichtung 26 gelegte Messprobe 20 (Prüfstapel) wird jetzt einer dynamischen Druckbeaufschlagung unterzogen. Es werden fünf Prüfzyklen gefahren. In jedem dieser kontinuierlich aneinander anschließenden Prüfzyklen wird die Messprobe bei einer Prüfgeschwindigkeit von 50 mm/min bis zu einem Druck von 200 N/100 cm² zusammengedrückt. Sofort nach Erreichen dieser Prüfkraft wird die Kraftwirkung mit der gleichen Prüfgeschwindigkeit bis zur Kraftanzeige Null des Prüfgeräts zurückgefahren und sodann wieder erhöht. Bei Erreichen der vorbestimmten Höchstprüfkraft von 200 N im fünften Prüfzyklus wird von dem Prüfgerät in diesem Zustand zugleich die Dicke der Messprobe bestimmt. Sie wird als $a_{30}$-5H-Dicke bezeichnet. Im Wesentlichen unmittelbar danach fährt die Prüfeinrichtung 26 den Messkopf 28 so weit zurück, dass sich die Messprobe unbehindert "erholen" kann. Nach 3 min wird manuell die Dicke, also die Stapelhöhe der Messprobe, wie vorausgehend beschrieben ermittelt. Sie wird als $a_{30}$-5E3-Dicke bezeichnet.

Das auf diese Weise ermittelte Rückstellvermögen wird wie folgt berechnet:

Dicke der Messprobe nach der Entspannung multipliziert mit 100% dividiert durch die Dicke der Messprobe vor der Belastung, also

$$\frac{(a_{30} - 5E3) \cdot 100\,\%}{a_{30}}$$

Die Messungen werden an fünf Messproben vorgenommen. Zur Dokumentierung des Ergebnisses werden die Vliesdicke des Wattebands sowie das eingangs ermittelte Flächengewicht des Wattebands in g/m² und das Rückstellvermögen in % angegeben.

Die nachfolgenden Tabellen zeigen Vergleichsmessungen bei erfindungsgemäßen und bekannten Wattebändern.

n bedeutet dabei die Anzahl der Messwerte. Die Messwerte sind angegeben als Mittelwert mit Standardabweichung.

Tabelle 1: Vertikale Flüssigkeitsaufnahme /Wasserhaltevermögen

| Prüfmerkmale | Einh | Watteband mit Mikrofaser | Watteband mit Mikrofaser | | Bel Nature | Bel Family | Ebelin (dm) - perforiert | n |
|---|---|---|---|---|---|---|---|---|
| | | 20% Mikrostapel -faser; 80% BW | 40% Mikrostapel -faser; 60% BW | | 100 % BW | 100 % Viskose | 100% BW | |
| **Vertikale Flüssigkeits- aufnahme:** | | | | | | | | |
| **Probengewicht** | g | 2,7 / 0,2 | 2,2 / 0,9 | | 3,0 / 0,2 | 3,3 / 0,2 | 2,3 / 0,2 | 20 |
| **Gesamtflüssig -keits- aufnahme** | g | 16,5 / 1,5 | 12,6 / 0,94 | | 18,7 / 1,2 | 15,9 / 1,6 | 15,9 / 1,6 | 20 |
| **Flüssigkeits- aufnahme spezifisch** | g/g | 6,0 / 0,4 | 5,7 / 0,4 | | 6,2 / 0,4 | 4,9 / 0,5 | 6,9 / 0,4 | ber. |
| **Steighöhe** | mm | **35,0/1,7** | **25,2 / 0,89** | | **42,0 / 7,9** | **46,3 / 3,2** | **57,0 / 2,9** | 20 |
| **Wasserhalte- vermögen:** | | | | | | | | |
| **Saugfähigkeit Drahtkörbchen** | g/g | 23,4 / 0,9 | 20,5/1,08 | | 21,8/1,0 | 18,1 / 0,5 | 22,1/0,8 | 20 |
| **Absinkdauer** | s | 8,8 / 0,8 | 8,6 / 0,9 | | 2,9 / 0,4 | 2,9 / 0,2 | 4,5 / 1,2 | 20 |
| Vertikale Flüssigkeitsaufnahme: 10 Minuten , 40 x 100 mm Längsrichtung | | | | | | | | |

Zwei erfindungsgemäße Wattebänder sind gebildet aus 80% Baumwollfasern und 20% Mikrostapelfasern aus Polyester bzw. aus 60% Baumwolle und 40% Mikrostapelfasern aus Polyester. Das Flächengewicht des jeweiligen Wattebands beträgt ca. 560 g/m$^2$; die Rohdichte liegt jeweils bei etwa 0,06 g/cm$^3$. Es wurde die vertikale Flüssigkeitsaufnahme und die Steighöhe sowie das Wasserhaltevermögen unter Verwendung der Drahtkörbchenmethode bestimmt. Als Vergleichsprodukte wurde ein Watteband der Marke "Bel Nature" aus 100% Baumwolle, "Bel Family" aus 100% Viskose und der Marke "Ebelin" (dm) aus 100% Baumwolle getestet.

Tabelle 1 zeigt, dass die erfindungsgemäßen Produkte eine vorteilhaftere Steighöhe aufweisen, die deutlich unter 40 mm liegt. Ein höherer Anteil von Mikrostapelfasern bewirkt eine Absenkung der Steighöhe.

Man erkennt außerdem, dass das Wasserhaltevermögen im Bereich von wenigstens 20 g/g, bestimmt durch die Drahtkörbchenmethode, hinreichend ist. Durch eine Erhöhung des Anteils der Mikrostapelfasern kann dies reduziert werden. Die hervorragenden Absinkdauern der erfindungsgemäßen Wattebänder erweisen sich als besonders vorteilhaft.

Tabelle 2: Rückstellvermögen

| Prüfmerkmale | Einheit | Watteband mit Mikrostapelfasern | Watteband mit Mikrostapelfasern | Watte ohne Mikrostapelfasern |
|---|---|---|---|---|
| | | 20% Mikrostapelfaser, 80 % Baumwolle | 37% Mikrostapelfaser, 63 % Baumwolle | 100 % Baumwolle |
| | | n = 7 | n=4 | n=4 |
| Probengewicht | g | 4,5 / 0,2 | 4,84 | 4,58 |

(fortgesetzt)

| Prüfmerkmale | Einheit | Watteband mit Mikrostapelfasern | Watteband mit Mikrostapelfasern | Watte ohne Mikrostapelfasern |
|---|---|---|---|---|
| Dicke der Messprobe | mm | 65,9 / 2,9 | 61,8 | 57 |
| Rohdichte | g/cm$^3$ | 0,007 / 0,0 | 0,008 | 0,008 |
| Rückstellvermögen | % | **52,2 / 1,8** | **55** | **50** |

Tabelle 2 verdeutlicht Messungen des Rückstellvermögens von erfindungsgemäßen Wattebändern mit 20% bzw. 37% Mikrostapelfasern und einem nicht erfindungsgemäßen Watteband aus 100% Baumwollfasern. Das Rückstellvermögen wird durch Zugabe von Mikrostapelfasern in vorteilhafter Weise beeinflusst und beträgt mehr als 50%.

Um einen Vergleich von Wattebändern mit einer Aussage über das Rückstellvermögen hinsichtlich der Faserzusammensetzung, unabhängig von ursprünglichem Flächengewicht und ursprünglicher Dichte machen zu können, wurden die Wattebänder auf eine einheitliche Dichte gebracht. Dazu wurden die Wattebänder nochmals mit einer Laborkarde auf die Rohdichte von ca. 0,008 g/cm$^3$ eingestellt.

**Patentansprüche**

1. Mehrfach auf sich selbst gefaltetes Watteband aus Baumwollfasern und/oder Viskosefasern oder anderen zellulosischen Fasern mit einem Anteil von 3 -80 Gew.-%, insbesondere von 3 - 50 Gew.-% Mikrostapelfasern, wobei das Watteband eine vertikale Steighöhe für eine wässrige Flüssigkeit von höchstens 40 mm aufweist.

2. Watteband nach Anspruch 1, **dadurch gekennzeichnet, dass** die vertikale Steighöhe 10 - 40 mm, insbesondere 15 - 38 mm beträgt.

3. Watteband nach Anspruch 1 oder 2, **dadurch gekennzeichnet, dass** der Anteil der Mikrostapelfasern 8 - 30 Gew.-% beträgt.

4. Watteband nach Anspruch 1, 2 oder 3, **dadurch gekennzeichnet, dass** die Mikrostapelfasern eine Länge von wenigstens 5 mm, insbesondere von wenigstens 7 mm aufweisen.

5. Watteband nach Anspruch einem oder mehreren der vorstehenden Ansprüche, **dadurch gekennzeichnet, dass** es sich bei den Mikrostapelfasern um Polyesterfasern (PES) handelt.

6. Watteband nach einem oder mehreren der vorstehenden Ansprüche, **dadurch gekennzeichnet, dass** das Flächengewicht des Wattebands im flachgelegten Zustand wenigstens 400 g/m$^2$ beträgt.

7. Watteband nach Anspruch 6, **dadurch gekennzeichnet, dass** das Flächengewicht des Wattebands im flachgelegten Zustand 400 g/m$^2$ - 800 g/m$^2$, insbesondere 450 g/m$^2$ - 750 g/m$^2$, insbesondere 500 g/m$^2$ - 650 g/m$^2$ beträgt.

8. Watteband nach einem oder mehreren der vorstehenden Ansprüche, **dadurch gekennzeichnet, dass** seine Dichte 0,01 - 0,1 g/m$^3$, insbesondere 0,02 - 0,07 g/m$^3$, insbesondere 0,03 - 0,06 g/m$^3$ beträgt.

9. Watteband nach einem oder mehreren der vorstehenden Ansprüche, **dadurch gekennzeichnet, dass** die Absinkdauer in wässriger Flüssigkeit wenigstens 3 s, insbesondere wenigstens 3,5 s, insbesondere wenigstens 4 s, und weiter insbesondere wenigstens 4,5 s beträgt.

10. Watteband nach einem oder mehreren der vorstehenden Ansprüche, **dadurch gekennzeichnet, dass** das Wasserhaltevermögen (Drahtkörbchenmethode) wenigstens 21 g/g, insbesondere wenigstens 24 g/g beträgt.

11. Watteband nach einem oder mehreren der vorstehenden Ansprüche, **dadurch gekennzeichnet, dass** das Rückstellvermögen größer als 50 %, insbesondere wenigstens 52 % beträgt.

12. Watteband nach einem oder mehreren der vorstehenden Ansprüche, **dadurch gekennzeichnet, dass** zusätzlich wärmeschmelzbare Bindefasern enthalten sind.

**13.** Watteband nach Anspruch 12, **dadurch gekennzeichnet, dass** die Bindefasern in einem gewichtsprozentualen Anteil von 5 - 20 Gew.-%, insbesondere 5 - 15 Gew.-% enthalten sind.

**14.** Watteband nach Anspruch 12 oder 13, **dadurch gekennzeichnet, dass** die Bindefasern Mehrkomponentenfasern, insbesondere Bikomponenten-Fasern sind.

**15.** Watteband nach Anspruch 14, **dadurch gekennzeichnet, dass** die Mehrkomponenten-Fasern eine Faserstärke von 1,3 - 10 dtex, insbesondere von 1,3 - 3,0 dtex aufweisen.

**16.** Watteband nach Anspruch 14 oder 15, **dadurch gekennzeichnet, dass** die Mehrkomponenten-Fasern eine Faserlänge von 3 - 60 mm aufweisen.

**17.** Watteband nach Anspruch 14, 15 oder 16, **dadurch gekennzeichnet, dass** es sich bei den Bikomponentenfasern um Copolyester (CO-PES) /Polyester (PES)- Bikomponentenfasern handelt.

**18.** Watteband nach einem der Ansprüche 12 - 17, **dadurch gekennzeichnet, dass** der Schmelzpunkt der wärmeschmelzbaren Bindefasern oder der niedrig schmelzenden Komponente der Mehrkomponentenfasern geringer ist als der Schmelzpunkt der Mikrostapelfasern.

6

10

2

**Fig. 1a**

4

12

14

8

**Fig. 1b**

6

15

**Fig. 1c**

**Fig. 2**

**Europäisches Patentamt**

# EUROPÄISCHER RECHERCHENBERICHT

Nummer der Anmeldung

EP 06 00 4886

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 521 877 B (PAUL HARTMANN AG) 13. April 2005 (2005-04-13) * Seite 2, Absatz 1 - Absatz 3 * * Seite 2, Absatz 8 - Seite 3, Absatz 20 * * Seite 4, Absatz 32 - Seite 5, Absatz 40 * ----- | 1-5,9-18 | INV. A61K8/02 ADD. A61F13/15 D04H1/42 |
| X | PATENT ABSTRACTS OF JAPAN Bd. 1998, Nr. 10, 31. August 1998 (1998-08-31) & JP 10 113222 A (TORAY IND INC), 6. Mai 1998 (1998-05-06) * Zusammenfassung * ----- | 1 | |
| X | EP 1 106 172 A (GEORGIA-PACIFIC FRANCE) 13. Juni 2001 (2001-06-13) * Seite 1, Absatz 1-3 * ----- | 1-7 | |
| X | EP 1 264 561 A (PAUL HARTMANN AKTIENGESELLSCHAFT) 11. Dezember 2002 (2002-12-11) * Spalte 4, Absatz 20 - Spalte 5, Absatz 23 * ----- | 1-5, 12-18 | |
| X | EP 0 851 052 A (PAUL HARTMANN AKTIENGESELLSCHAFT) 1. Juli 1998 (1998-07-01) * Spalte 3, Zeile 24 - Zeile 31 * ----- | 1-4 | RECHERCHIERTE SACHGEBIETE (IPC) A61K A61F D04H |
| P,X | EP 1 630 276 A (PAUL HARTMANN AG) 1. März 2006 (2006-03-01) * Seite 2, Absatz 2 * * Seite 3, Absatz 13 - Absatz 15 * ----- | 1-5,9-18 | |

Der vorliegende Recherchenbericht wurde für alle Patentansprüche erstellt

| Recherchenort | Abschlußdatum der Recherche | Prüfer |
|---|---|---|
| München | 29. August 2006 | Settele, U |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**  EP 06 00 4886

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-08-2006

| Im Recherchenbericht angeführtes Patentdokument | | Datum der Veröffentlichung | Mitglied(er) der Patentfamilie | | Datum der Veröffentlichung |
|---|---|---|---|---|---|
| EP 1521877 | B | 14-12-2005 | EP | 1521877 A2 | 13-04-2005 |
| JP 10113222 | A | 06-05-1998 | KEINE | | |
| EP 1106172 | A | 13-06-2001 | AU | 2183801 A | 18-06-2001 |
| | | | CA | 2393205 A1 | 14-06-2001 |
| | | | DE | 1106172 T1 | 25-10-2001 |
| | | | WO | 0141731 A2 | 14-06-2001 |
| | | | PL | 356052 A1 | 14-06-2004 |
| EP 1264561 | A | 11-12-2002 | AT | 330505 T | 15-07-2006 |
| | | | DE | 10127514 A1 | 30-01-2003 |
| EP 0851052 | A | 01-07-1998 | AT | 215629 T | 15-04-2002 |
| | | | DE | 19654457 A1 | 02-07-1998 |
| EP 1630276 | A | 01-03-2006 | DE | 102004042896 A1 | 02-03-2006 |

EPO FORM P0461

Für nähere Einzelheiten zu diesem Anhang : siehe Amtsblatt des Europäischen Patentamts, Nr.12/82